**Europäisches Patentamt**

(19) **European Patent Office**

**Office européen des brevets**

(11) Veröffentlichungsnummer: **0 230 937**
**B1**

(12) **EUROPÄISCHE PATENTSCHRIFT**

(45) Veröffentlichungstag der Patentschrift: **27.12.90**

(21) Anmeldenummer: **87100575.7**

(22) Anmeldetag: **17.01.87**

(51) Int. Cl.⁵: **A 61 B 17/08, A 61 F 2/08**

(54) **Medizinische Agraffe.**

(30) Priorität: **28.01.86 CH 320/86**

(43) Veröffentlichungstag der Anmeldung:
**05.08.87 Patentblatt 87/32**

(45) Bekanntmachung des Hinweises auf die
Patenterteilung:
**27.12.90 Patentblatt 90/52**

(84) Benannte Vertragsstaaten:
**AT DE FR GB IT**

(56) Entgegenhaltungen:
**EP-A-0 033 641**
**FR-A-2 213 761**
**GB-A-2 118 474**
**US-A-1 409 813**
**US-A-2 839 815**
**US-A-3 741 205**
**US-A-4 263 903**

(73) Patentinhaber: **GEBRÜDER SULZER**
**AKTIENGESELLSCHAFT**
**Zürcherstrasse 9**
**CH-8401 Winterthur (CH)**

(72) Erfinder: **Frey, Otto**
**Wallrütistrasse 56**
**CH-8400 Winterthur (CH)**
Erfinder: **Koch, Rudolf**
**Oberdorfstrasse 229**
**CH-8267 Berlingen (CH)**

(74) Vertreter: **Sparing Röhl Henseler Patentanwälte**
**European Patent Attorneys**
**Rethelstrasse 123**
**D-4000 Düsseldorf 1 (DE)**

Courier Press, Leamington Spa, England.

**Beschreibung**

Die Erfindung betrifft eine medizinische Agraffe, vorzugsweise zum Fixieren von künstlichen Bändern und Sehnen an einem Knochen, die zwei mit einer in Ausziehrichtung widerlagerartigen Struktur versehene, dornartige Verankerungsstifte enthält, die von einem verbindenden Brückenelement überbrückt sind.

Eine Agraffe der vorstehend beschriebenen Art ist beispielsweise bekannt aus der US—PS 4,263,903; sie besteht in einem schmalen Steg, der an den Enden zwei dornartigen Verankerungsstifte aufweist, die abgewinkelt sind. Die Fixierung beispielsweise eines künstlichen Bandes oder einer künstlichen Sehne mit Hilfe dieser Agraffe, deren Dorne in einen Knochen eingetrieben werden, erfolgt nur durch einen Klemmeffekt über die relativ schmale Stegbreite. Weiterhin bilden die Verankerungsdorne und der Steg eine unlösbare Einheit. Daher sind für unterschiedliche "Eindringtiefen" der dornenartigen Stifte in den Knochen eine Anzahl von Einzelelementen notwendig, die sich nur in der Länge ihrer Verankerungsdorne unterscheiden. Will man dem Operateur ein ausreichendes Angebot mit unterschiedlichen Steglängen- und -breiten sowie für jede dieser Längen und Breiten verschiedene Dornlängen zur Verfügung stellen, so ergibt sich ein grosses Sortiment, das die Fabrikation und die Lagerhaltung erschwert und einen grossen Aufwand erfordert.

Aufgabe der Erfindung ist es daher, eine Agraffe zu schaffen, bei der eine Sehne oder ein Band relativ grossflächig gehalten und die Anzahl zu einem vollständigen Satz gehörender Einzelelemente möglichst reduziert sind.

Mit der vorliegenden Erfindung wird diese Aufgabe dadurch gelöst, dass das Brückenelement aus einer individuell anpassbaren bleibend verformbaren Metallplatte mit laschenartigen Ansätzen besteht, in denen die Verankerungsstifte lösbar befestigt sind.

Eine bleibend verformbare Metallplatte bietet statt einem linienartigen Festklemmen eines Bandes einen über eine grössere Fläche verteilten Klemmeffekt; ihre bleibende Verformbarkeit ermöglicht darüberhinaus eine Anpassung ihrer Fläche an die individuelle Knochenform. Weiterhin können — beispielsweise nach einer Röntgenaufnahme, mit der die Knochendicke ermittelt worden ist — aus einem Sortiment von verschieden langen Verankerungsstiften diejenigen mit einer geeigneten Länge individuell ansgewählt und mit der Platte verbunden werden, ehe diese einer Sterilisation unterzogen wird. Dadurch kann das Sortiment an Plattengrössen und Stiftlängen, die zu einem vollständigen Bausatz gehören, relativ klein gehalten werden.

Eine vorteilhafte Möglichkeit für die Verbindung der Verankerungsstifte und der Platte ergibt sich, wenn die mit einem Kopf versehenen Verankerungsstifte in hohle, aus den laschenartigen Ansätzen vorstehende Gewindeösen eingeschraubt sind, so dass sie die Laschenfläche knaufartig überragen. Die Ausbildung von vorstehenden Knaufen kann dabei für den Eingriff eines Ein- und Ausschlaginstrumentes dienen, das mit Vorteil einen stempelartigen Schaft, der in einer gabelartigen Erweiterung endet, und weiterhin in der gabelartigen Erweiterung kreisringsektorartige Ausnehmungen mit T-förmigem Profil für die Aufnahme der knaufartigen Vorsprünge der Agraffe aufweist, wobei schliesslich noch Mittel vorgesehen sind, mit denen die knaufartigen Vorsprünge der Agraffe und das Instrument relativ zueinander fixierbar sind.

Die genannten Mittel können dabei z.B. in einer in Umfangsrichtung der Kreisringabschnitte geneigten Decke der Ausnehmungen oder in Klemmschrauben bestehen mit denen die Köpfe der Verankerungsstifte in dem Instrument fixiert sind.

Bit einer weiteren vorteilhaften Ausführungsform der Agraffe kann die Metallplatte über das durch die Laschenunterseiten im Bereich der Verankerungsstifte gegebene Auflagenniveau brückenbogenartig erhöht und mit Haftnägeln versehen sein, die bis zu einer Tiefe von mindestens 1/3 der Länge der Verankerungsstifte unter das Auflageniveau reichen. Durch diese ebenfalls in den Knochen eindringenden Haftnägel wird eine erhöhte Haftung des Bandes erreicht, da die Nägel als zusätzliche, das zu haltende Band durchsetzende Anker wirken.

Im folgenden wird die Erfindung anhand eines Ausführungsbeispiels im Zusammenhang mit der Zeichnung näher erläutert.

Fig. 1 zeigt in einem Schnitt I—I von Fig. 2 ein Ausführungsbeispiel der neuen Agraffe mit einem zusätzlich andeutungsweise skizzierten Einschlaginstrument;

Fig. 2 ist eine Aufsicht auf Fig. 1 ohne Instrument;

Fig. 3 ist der Schnitt III—III von Fig. 1;

Fig. 4 gibt — teiltweise im Schnitt — ein Ein- und Ausschlaginstrument wieder;

Fig. 5 ist, vergrössert, eine Ansicht des Instrumentes nach Fig. 4 von unten mit schematisch angedeuteter Agraffe;

Fig. 6 schliesslich stellt im gleichen Schnitt wie Fig. 3 eine Möglichkeit für die Fixierung der Agraffe an dem Einschlaginstrument dar.

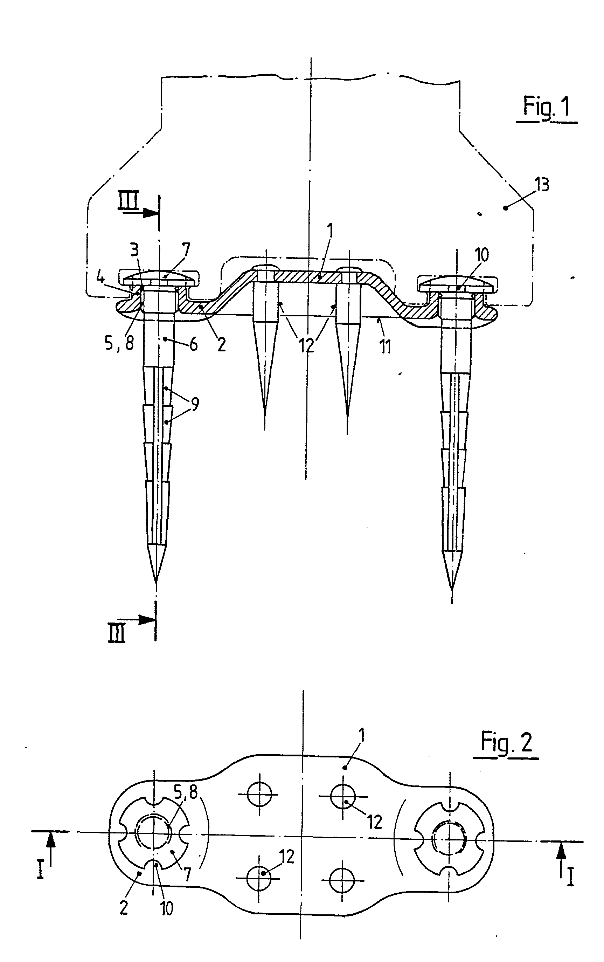

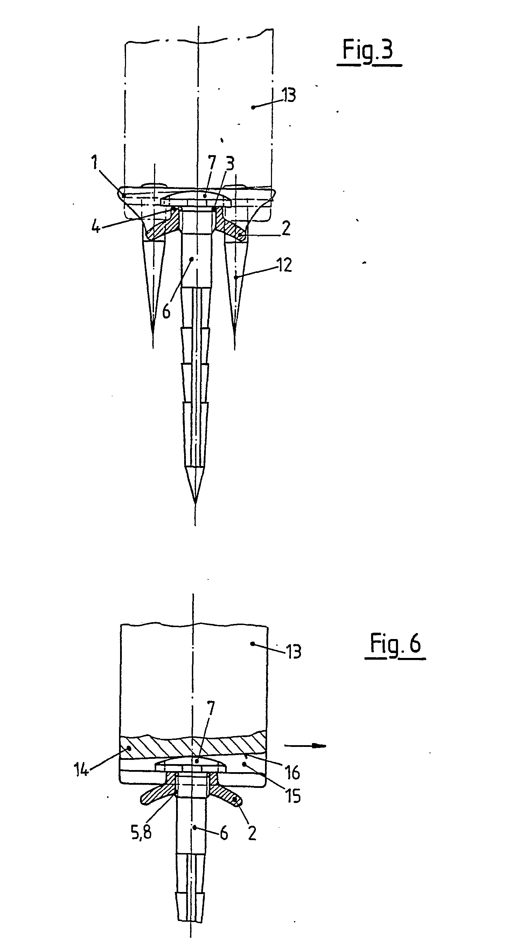

Die Metallplatte 1 der als Ausführungsbeispiel gezeigten Agraffe ist im wesentlichen quadratisch (Fig. 2) und weist an zwei einander gegenüberliegenden Seiten je einen laschenartigen Ansatz 2 auf. Jede Lasche 2 besitzt eine Oese 3, die von einem nach oben aus ihrer Oberfläche hervorstehenden Rand 4 umgeben ist; in den Rand 4 ist ein Gewinde 5 eingeschnitten. Die Flanken der Laschen 2, die ebenso wie die Metallplatten 1 zur Anpassung an einen individuellen Knochen bleibend verformbar sind, sind leicht nach unten abgebogen (Fig. 3), so dass sie sich beispielsweise an die Rundung eines Röhrenknochens anschmiegen können.

In die Gewinde 5 sind Verankerungsstifte 6 eingeschraubt, die für diese lösbare Verbindung der Metallplatte 1 unmittelbar unter ihrem Kopf mit einem entsprechenden Gewinde 8 versehen sind. Sie haben die Form eines langen Dornes, der in Ausziehrichtung eine widerlagerartige Struktur 9 besitzt. Für den Ansatz eines Werkzeugs weist ihr Kopf 7 auf seinem Umfang Ausnehmungen 10 auf. Mit den Unterseiten in ihrem Scheitelbereich definieren die Laschen 2 ein Auflagenfveau 11 (Fig. 1).

Gegenüber diesem Auflagenfveau 11 ist die Metallplatte 1 zur Aufnahme des nicht gezeigten künstlichen Bandes brückenbogenartig erhöht. In ihrem "Brückenbogen" sind Haftnägel 12 vorgesehen, deren Länge so gewählt ist, dass die das Auflagenfveau 11 um mindestens 1/3 der Länge der Verankerungsstifte 6 nach unten überragen.

In den Fig. 1 und 3 ist ein Ein- und Ausschlaginstrument 13 angedeutet, in dem die Agraffe für ihre "Montage" und "Demontage" in bzw. aus dem Knochen fixierbar ist.

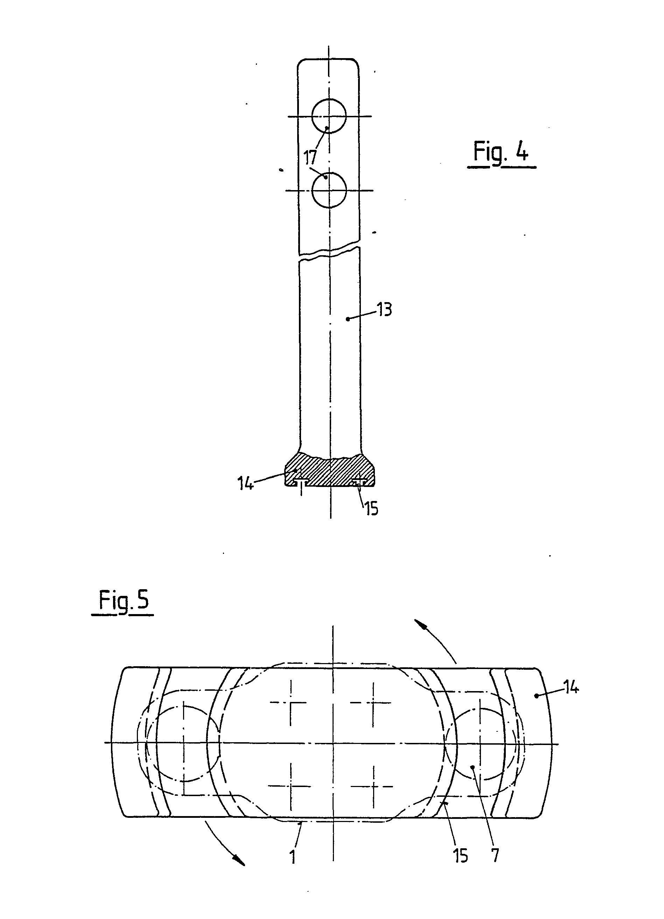

Das Instrument 13 besteht aus einem stempelartigen Schaft, der sich unten gabelförmig erweitert und in diese Erweiterung 14 zwei in Querschnitt T-förmige Ausnehmungen oder Nuten 15 hat, die einen Sektor eines Kreisringes bilden (Fig. 5). In diesen Nuten 15, die einen Abstand gleich demjenigen der Verankerungsstifte 6 der Agraffe haben, können die knaufartig vorstehenden Köpfe der Stifte 6 eingreifen. Sie werden durch eine geneigt verlaufende Decke 16 im Inneren der Nut 15 in den horizontalen "Balken" des T-Querschnittes durch eine Relativdrehung zwischen dem Instrument 13 und der Agraffe eingeklemmt und fixiert. Selbstverständlich sind auch andere Arten der Fixierung zwischen Instrument 13 und Agraffe möglich, wie z.B. ein Festklemmen der Agraffe mit Hilfe einer Klemmschraube.

Das Einschlagen der Agraffe erfolgt durch Schläge auf den stempelartigen Schaft 13, während für ein Ausschlagen durch eine der Bohrungen 17 im oberen Bereich des Stempels 13 nicht gezeigte Querstäbe gesteckt werden, gegen die dann von unten nach oben gaschlagen wird.

## Patentansprüche

1. Medizinische Agraffe, vorzugsweise zum Fixieren von künstlichen Bändern und Sehnen an einem Knochen, die zwei mit einer in Ausziehrichtung widerlagerartigen Struktur (9) versehene, dornartige Verankerungsstifte (6) enthält, die von einem verbindenden Brückenelement überbrückt sind, dadurch gekennzeichnet, dass das Brückenelement aus einer individuell anpassbaren bleibend verformbaren Metallplatte (1) mit laschenartigen Ansätzen (2) besteht, in denen die Verankerungsstifte (6) lösbar befestigt sind.

2. Agraffe nach Anspruch 1, dadurch gekennzeichnet, dass die mit einem Kopf (7) versehenen Verankerungsstifte (6) in hohle, aus den laschenartigen Ansätzen (2) vorstehende Gewindeösen (3) eingeschraubt sind, so dass sie die Laschenfläche knaufartig überragen.

3. Agraffe nach Anspruch 1 oder 2, dadurch gekennzeichnet, dass die Metallplatte (1) über das durch die Laschenunterseiten im Bereich der Verankerungsstifte (6) gegebene Auflagenfveau (11) brückenbogenartig erhöht und mit Haftnägeln versehen ist, die bis zu einer Tiefe von mindestens 1/3 der Länge der Verankerungstifte (6) unter das Auflagenfveau (11) reichen.

4. Ein- und Ausschlaginstrument (13) für eine Agraffe nach einem der Ansprüche 2 oder 3, mit einem stempelartigen Schaft, der in einer gabelartigen Erweiterung (14) endet, gekennzeichnet durch in der gabelartigen Erweiterung (14) vorgesehene kreisringsektorartige Ausnehmungen (15) mit T-förmigem Profil für die Aufnahme der knaufartigen Vorsprünge (4, 7) der Agraffe und durch Mittel (16), mit denen die knaufartigen Vorsprünge (4, 7) der Agraffe und das Instrument (13) relativ zueinander fixierbar sind.

5. Instrument nach Anspruch 4, dadurch gekennzeichnet, dass die Mittel in einer in Umfangsrichtung der Kreisringabschnitte geneigten Decke (16) der Ausnehmungen (15) bestehen.

## Revendications

1. Agrafe à usage médical, de préférénce pour la fixation de ligaments et de tendons artificiels sur un os, qui comporte deux pointes d'ancrage (6) en forme d'épines, à structure (9) jouant le rôle de butée dans le sens d'extraction, qui sont pontées par un élément de jonction, caractérisée en ce que l'élément en pont est constitué d'une plaque métallique (1) individuellement adaptable et à capacité de déformation permanente, présentant des appendices (2) formant des pattes dans lesquelles les pointes d'ancrage sont fixées de manière amovible.

2. Agrafe selon la revendication 1, caractérisée en ce que les pointes d'ancrage (6), munies d'une tête (7), sont vissées dans des oeillets filetés (3) qui font saillie des appendices (2) formant des pattes de telle sorte qu'ils dépassent de la surface des pattes à la manière de boutons.

3. Agrafe selon la revendication 1 ou 2, caractérisée en ce que la plaque métallique (1) est relevée en arche de pont au dessus du niveau d'appui (11) déterminé par les faces inférieures des pattes dans la région des pointes d'ancrage (6), et est munie de clous de fixation qui s'étendent en dessous du niveau d'appui (11) jusqu'à une profondeur atteignant au moins le tiers de la longueur des pointes d'ancrage (6).

4. Instrument (13) destiné à enfoncer et à extraire une agrafe selon l'une des revendications 2 ou 3, avec un corps en forme de poinçon qui se termine par une patie (14) élargie en fourche, caractérisé par des évidements (15) en forme de secteurs d'anneau circulaire et à profil en T prévus dans la partie (14) élargie en fourche pour recevoir les saillies en boutons (4, 7) de l'agrafe, et par des moyens (16) à l'aide desquels les saillies en boutons (4, 7) de l'agrafe et l'instrument (13) peuvent être fixés en position relative.

5. Instrument selon la revendication 4, caractérisé en ce que lesdits moyens consistent un une couverture (16) des évidements (15) qui est inclinée dans le sens circonférentiel des secteurs d'anneau circulaire.

**Claims**

1. A medical clamp, preferably for securing artificial ligaments and sinews to a bone, containing two spike-like anchoring pins (6) having an abutment-like structure (9) in the pull-out direction and connected by a bridging element, characterised in that the bridging element is an individually adaptable, permanently deformable metal plate (1) having lug-like extensions (2) in which the anchoring pins (6) are releasably fastened.

2. A clamp according to claim 1, characterised in that the anchoring pins (6), which have a head (7), are screwed into hollow threaded eyelets (3) projecting from the lug-like extensions (2), so that the pins project like knobs above the surface of the lugs.

3. A clamp according to claim 1 or 2, characterised in that the metal plate (1) is raised above the bearing level (11) defined by the undersides of the lugs in the neighbourhood of the anchoring pins (6) after the style of a curved bridge and provided with nails which extend to a depth of at least one-third of the length of the anchoring pins (6) below the bearing level (11).

4. A knocking-in and knocking-out instrument (13) for a clamp according to claim 2 or 3, comprising a punch-like shank ending in a forked extension (14), characterised by circular sector-shaped recesses (15) formed in the forked extension (14) and having a T-shaped cross-section for receiving the knob-like projections (4, 7) of the clamp, and by means (16) for securing the knob-like projections (4, 7) of the clamp relative to the instrument (13).

5. An instrument according to claim 4, characterised in that the means comprise a cover (16) over the recesses (15) and inclined in the peripheral direction of the circular portions.

4

EP 0 230 937 B1

Fig. 1

Fig. 2

1

Fig.3

Fig.6

Fig. 4

17

13

14

15

Fig. 5

14

1

15   7